# EUROPÄISCHE PATENTSCHRIFT

(11) **EP 2 465 545 B1**
(45) Veröffentlichungstag und Bekanntmachung des Hinweises auf die Patenterteilung: **27.05.2015**
(21) Anmeldenummer: 11193888.2
(22) Anmeldetag: 15.12.2011
(51) Int. Cl.: A61L 2/22, A61L 2/18, B65B 55/02, B67C 7/00

(54) **Vorrichtung zum Sterilisieren von Behältnissen**
Device for sterilising containers
Dispositif de stérilisation de récipients

(30) Priorität: 16.12.2010 DE 102010054788
(43) Veröffentlichungstag der Anmeldung: 20.06.2012
(73) Patentinhaber: Krones AG, 93073 Neutraubling (DE)
(72) Erfinder: Peter, Michael, 93051 Regensburg (DE); Weber, Michael, 93177 Altenthann (DE); Hackl, Sascha, 93128 Regenstauf (DE); Morawe, Thomas, 93092 Barbing (DE)
(74) Vertreter: Hannke, Christian

(56) Entgegenhaltungen:
- EP-A1- 2 052 744
- EP-A1- 2 279 952
- WO-A1-01/44053
- WO-A1-02/15946
- WO-A1-99/01374
- WO-A1-2010/052068
- WO-A1-2011/111513
- GB-A- 2 271 347
- US-A- 4 152 464
- US-A- 5 879 648

## Beschreibung

Die vorliegende Erfindung bezieht sich auf eine Vorrichtung zum Sterilisieren von Behältnissen. Aus dem Stand der Technik sind Anlagen bekannt, welche Behältnisse, beispielsweise Kunststoffbehältnisse, mit Getränken befüllen. Dabei ist es ebenfalls bekannt, dass derartige Fülleinrichtungen Sterilisationseinrichtungen angeschlossen sind, welche die abzufüllenden Behältnisse desinfizieren und insbesondere eine Außendesinfektion dieser Getränkebehälter vornehmen. Dabei werden die Behältnisse durch flaschenabhängig gestaltete Trägersysteme durch eine Behandlungskammer geführt.

EP2052744 beschreibt eine Vorrichtung zur Flaschendesinfektion mittel eingedüster Peroxidlösung.

WO0144053 offenbart eine aseptisch arbeitende Verpackungsmaschinen und ein Verfahren zu deren Vorsterilisation. Für das gemeinsame Eindüsen von Dampf und Sterilisationsmittel an einer einzigen Stelle in einen Sterilluftzufuhrkanal, der in den Sterilraum der Verpackungsmaschine einmündet, ist im Luftzufuhrkanal unmittelbar stromab hinter einem Sterilluftfilter eine Mischdüse angeordnet.

Üblicherweise weisen derartige Sterilisationseinheiten vergleichsweise große Sterilisationskammern auf, durch welche die Behältnisse geführt werden. Durch das große vorhandene Volumen innerhalb dieses Behandlungsraums oder Isolatorraums ist es nur schwer möglich, eine mit Dämpfen angereicherte Atmosphäre zu erreichen. Derzeit werden die Behältnisse mit vereinzelt angeordneten Düsen gezielt besprüht, wobei diese Düsen an in dem Isolatorraum verlegten Rohren montiert sind. In dieser Anordnung ist eine dauerhafte Benetzung der Behältnisse nicht möglich. Daneben ist auch die Justierung dieser Düsen sehr zeitintensiv und es kann durchaus zu Fehleinstellungen kommen.

Demzufolge liegt der vorliegenden Erfindung die Aufgabe zugrunde, eine Vorrichtung zum Sterilisieren von Behältnissen zu schaffen, welche die Erzeugung einer mit Dämpfen angereicherten Atmosphäre erleichtert. Daneben liegt der vorliegenden Erfindung die Aufgabe zugrunde, die Sterilisation von Behältnissen effizienter zu gestalten.

Die wird erfindungsgemäß durch eine Vorrichtung nach Anspruch 1 erreicht. Vorteilhafte Ausführungsformen und Weiterbildungen sind Gegenstand der Unteransprüche.

Eine Vorrichtung zum Behandeln von Behältnissen weist ein Gehäuse auf sowie eine innerhalb des Gehäuses und bevorzugt auch durch Wandungen in dieses Gehäuses ausgebildete Sterilisationskammer zum Sterilisieren der Behältnisse. Weiterhin weist die Vorrichtung eine Transporteinrichtung auf, welche die Behältnisse entlang eines vorgegebenen Transportpfades durch diese Sterilisationskammer transportiert. Daneben ist wenigstens eine Beaufschlagungseinrichtung vorgesehen, welche die Behältnisse während ihres Transportes durch die Sterilisationskammer mit einem fließfähigen Sterilisationsmedium beaufschlagt.

Erfindungsgemäß ist dabei die Beaufschlagungseinrichtung wenigstens teilweise in eine Wandung der Sterilisationskammer integriert. Bei dem fließfähigen Sterilisationsmedium handelt es sich insbesondere um eine Sterilisationsflüssigkeit und besonders bevorzugt um eine Sterilisationsflüssigkeit, welche beispielsweise Peressig - Säure oder Wasserstoffperoxid enthält.

Vorzugsweise handelt es sich bei der Beaufschlagungseinrichtung um eine oder mehrere Düsen, welche die Behältnisse mit dem Sterilisationsmedium beaufschlagen. Die Düsen sind dabei direkt in eine Wandung bzw. Einhausung der Sterilisationskammer eingebaut. Auf diese Weise müssen innerhalb des Sterilisationsraumes keine unnötigen Leitungen verlegt sein, welche das Sterilisationsmittel zu den Beaufschlagungseinrichtungen transportieren. Bevorzugt sind mehrere Beaufschlagungseinrichtungen bzw. Düsen vorgesehen und diese sind besonders bevorzugt derart angeordnet, dass sie die Behältnisse von unten und von der Seite mit dem Sterilisationsmedium beaufschlagen. Damit dient die Vorrichtung insbesondere zur Aussenentkeimung der Behältnisse.

Vorteilhaft ist die Sterilisationskammer wenigstens abschnittsweise kanalartig um den Transportpfad ausgebildet. Während im Stand der Technik die Behältnisse üblicherweise durch einen Behandlungsraum mit einem sehr großen Volumen befördert werden, wird hier vorgeschlagen, dass die Sterilisationskammer mit vergleichsweise geringem Volumen ausgeführt ist, damit eine effiziente Durchsetzung mit dem Sterilisationsmedium innerhalb der Sterilisationskammer möglich ist.

Vorteilhaft weist die Vorrichtung eine Abführeinrichtung zum Abführen eines gasförmigen Mediums aus der Sterilisationskammer auf. Dabei ist es beispielsweise möglich, dass beispielsweise zur Montage und zu Reinigungszwecken in sehr kurzer Zeit Sterilisationsmedium gemeinsam mit einem Trägermedium wie beispielsweise Sterilluft über die besagte Abführeinrichtung aus der Kammer abgeführt werden kann. Vorzugsweise ist dabei ein gezieltes Absaugen des in der Kammer befindlichen gasförmigen Mediums, welches beispielsweise mit Sterilisationsmittel versetzt sein kann, möglich.

Eine entsprechende Atmosphäre in der Sterilisationskammer kann durch eine systematisch angeordnete Absaugung gezielt in normalen Zustand gebracht werden um so einen schnelleren Maschineneingriff zu ermöglichen. Dabei wird der Luftstrom in diesem Fall teilweise wenn möglich ganz durch die Sterilisationskammer geführt und abgesaugt. Während eines Arbeitsbetriebes bzw. einer Produktion ist die Absaugung abgeschaltet bzw. die Abführeinrichtung geschlossen. Vorteilhaft wird hier zusätzlich das Düsensystem bzw. die Beaufschlagungseinrichtungen mit chemiefreiem Wasser beaufschlagt um so ebenfalls die chemischen Dämpfe zu binden oder niederzuschlagen und die mit Desinfektionsmitteln benetzten Oberflächen abzuspülen. Es wäre dabei auch möglich, mehrere der hier beschriebenen Sterilisationskammern hintereinander vorzusehen, welche hintereinander von den Behältnissen durchlaufen werden.

Bei einer weiteren vorteilhaften Ausführungsform weist die Vorrichtung auch eine Zuführeinrichtung für das gasförmige Medium auf und diese Zuführeinrichtung ist bevorzugt von der Abführeinrichtung beanstandet. Auf diese Weise kann durch eine gezielte Zuführung eines gasförmigen Mediums eine gerichtete Strömung innerhalb des Sterilisationsraums erreicht werden, falls eine Absaugung der Sterilisationsmedien gewünscht ist. Vorzugsweise ist in dem Gehäuse wenigstens eine verschließbare Montageöffnung angeordnet. Mit anderen Worten ist die Sterilisationskammer vorteilhaft mit Revisionsfenstern versehen, um einen Maschineneingriff zu ermöglichen.

Bei einer weiteren vorteilhaften Ausführungsform weist die Transporteinrichtung einen drehbaren Träger auf. Dabei ist es möglich, dass eine Sternsäule vorgesehen ist, auf der das Trägersystem montiert ist und um welche das Trägersystem rotiert. Dabei kann die Abstützung der besagten Sternsäule so gestaltet sein, dass die Flasche an einer Wandung bzw. einer Seite vorbei rotieren kann. Auf diese Weise ist das nicht benötigte Raumvolumen soweit wie möglich ausgegrenzt. Die Sternsäule kann dabei gleichzeitig auch die Aufnahme des Trägersystems und eine Wand oder Einhausung der Kammer sein. Bei dieser Ausführungsform wird die Sterilisationskammer also durch ein gegenüber dem Trägersystem stationäres Gehäuse eingehaust.

Vorteilhaft ist an dem Träger eine Vielzahl von Auslegern angeordnet, an denen jeweils Behältnisse führbar sind. So können beispielsweise an den Enden dieser Ausleger Greifelemente bzw. Greifklammern angeordnet sein, welche die Behältnisse während dieses Transportes halten. Daher werden bei dieser Ausführungsform die Behältnisse entlang einer Kreisbahn durch die Sterilisationskammer gefördert. Vorteilhaft weist ein Abschnitt der Transporteinrichtung eine die Sterilisationskammer abschließende Wandung auf. Dabei kann es sich beispielsweise um einen Teil der oben erwähnten Sternsäule handeln. Auch durch diese Vorgehensweise ist es möglich, die Sterilisationskammer bzw. den Sterilisationsraum klein zu halten.

Vorteilhaft weist die Sterilisationskammer einen unterhalb des Transportpfades ausgebildeten Sammelraum zum Sammeln des schließfähigen Mediums auf. So kann beispielsweise durch die konstruktive Ausführung der Sterilisationskammer deren Boden als Sammelbehälter genutzt werden. Das Sterilisationsmittel bzw. die Flüssigkeit wird aufgefangen und vorzugsweise wieder in einen Behandlungskreislauf eingespeist. Durch den geringen Behandlungsraum wird die Luft mit den Dämpfen der Behandlungsflüssigkeit aufkonzentriert wodurch die Behandlung und Desinfektion der Behältnisse zusätzlich intensiviert wird. Vorteilhaft sind die Beaufschlagungseinrichtungen bzw. die Behandlungsdüsen so angeordnet, dass der oder die Behälter seitlich dauerhaft mit diesen Desinfektionsmitteln beaufschlagt werden um so eine möglichst dauerhafte Behandlung durchzuführen. Auch wäre es möglich, in bestimmten Bereichen der Anlage eine höhere Anzahl an Düsen vorzusehen als in anderen Bereichen.

Dabei sind vorteilhaft die Düsen zum Auftragen des Desinfektionsmittels derart ausgelegt, dass die Tröpfchen so groß sind, um eine gewisse mechanische Wirkung zu erzielen, und gleichzeitig aber auch einen Nebel zu erzeugen, der die Atmosphäre mit Dämpfen anreichert. Dieser Nebel kann sich auf den Behältnissen niederschlagen und weist ebenfalls eine entkeimende Wirkung auf. Für kritische Bereiche der Behältnisse können zusätzliche Beaufschlagungseinrichtungen bzw. Düsen angebracht sein, um diese gezielt zu besprühen.

Bei einer weiteren vorteilhaften Ausgestaltung ist das Gehäuse kuppelartig ausgeführt. Mit anderen Worten kann die Sterilisationskammer eine Kuppel bzw. Abdeckung aufweisen, die in gerundeter Ausführung vorliegt. Diese gewölbten Abgrenzungen ermöglichen es, dass das Raumvolumen in der Behandlungskammer so gering wie möglich gehalten wird. Weiterhin kann diese kuppelartige Ausführungsform die Behandlungsflüssigkeit daran hindern, in die Behältnisse einzudringen, da die aufgesprühten Tropfen an der gewölbten Wandung direkt nach unten ablaufen und so ein direktes Eintropfen in die Behältnisse verhindert wird.

Wie oben erwähnt, weist die Vorrichtung vorteilhaft Rohrleitungen auf, welche die Beaufschlagungseinrichtungen mit dem fließfähigen Medium versorgen und diese Rohrleitungen sind dabei im Wesentlichen außerhalb der Sterilisationskammer angeordnet. So kann die Größe der Sterilisationskammer weiter verringert werden. Bei einer weiteren vorteilhaften Ausführungsform weist die Vorrichtung eine Zufuhröffnung zum Zuführen der Behältnisse und eine Abführöffnung zum Abführen der Behältnisse auf.

Dabei sind diese Zu- und Abführöffnungen so klein als möglich und so groß als nötig gestaltet um einerseits das Ein- und Austreten der Behältnisse in die bzw. aus der Sterilisationskammer zu gewährleisten und andererseits Gaseintritte und -austritte in die und aus der Sterilisationskammer soweit als möglich zu vermeiden. Auf diese Weise wird die Sterilisationskammer so gestaltet, dass das Behandlungssystem zur restlichen Anlage hin abgeschlossen ist.

Weitere vorteilhafte Ausführungsformen ergeben sich aus den beigefügten Zeichnungen:
Darin zeigen:
   - Fig. 1: Eine schematische Schnittdarstellung einer erfindungsgemäßen Vorrichtung zum Sterilisieren von Behältnissen;
   - Fig. 2: eine Draufsicht auf eine erfindungsgemäße Vorrichtung;
   - Fig. 3: eine weitere Draufsicht auf eine erfindungsgemäße Vorrichtung;
   - Fig. 4: eine Darstellung ähnlich der in Fig. 3 gezeigten Darstellung;
   - Fig. 5: eine Darstellung des Innenraums der Vorrichtung;
   - Fig. 6: eine weitere Darstellung des Innenraums der Vorrichtung;
   - Fig. 7: eine weitere Detaildarstellung der in Fig. 6 gezeigten Vorrichtung; und
   - Fig. 8: eine Darstellung der erfindungsgemäßen Anlage.

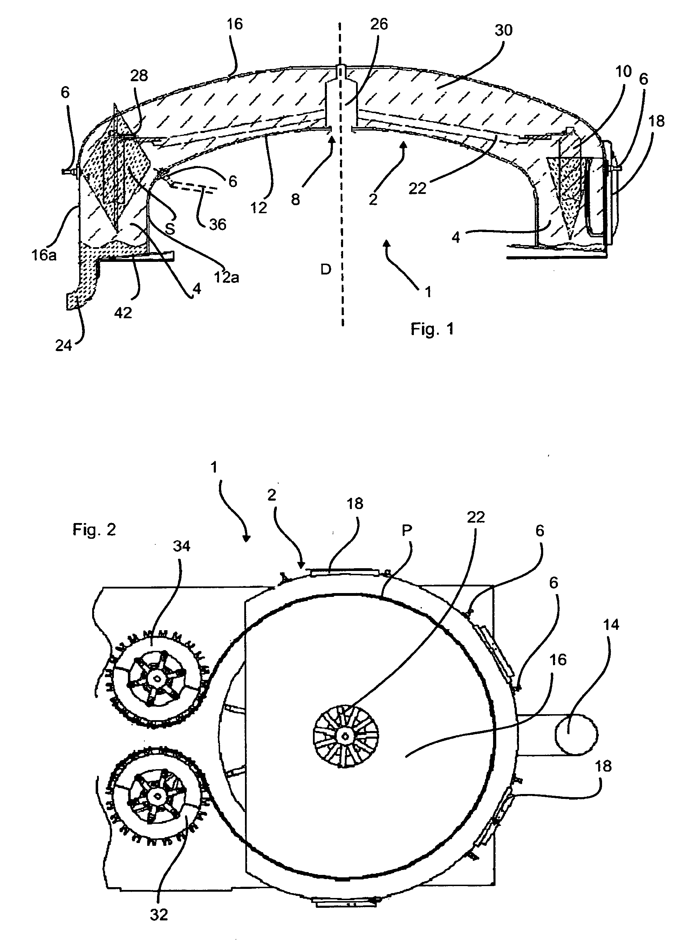

Fig. 1 zeigt eine schematische Schnittdarstellung einer erfindungsgemäßen Vorrichtung 1. Diese Vorrichtung 1 weist dabei einen um eine Drehachse D drehbaren Träger 26 auf, an dem eine Vielzahl von Auslegern 22 angeordnet ist, wobei an diesen Auslegern 22 wiederum Greifelemente 28 zum Halten von Behältnissen 10 angeordnet sind. Diese Greifelemente greifen dabei die Behältnisse 10 unterhalb der Mündung. Durch die so in der Gesamtheit mit 8 gekennzeichnete Transporteinrichtung werden hier die Behältnisse 10 entlang einer kreisförmigen Bahn um die Drehachse D gefördert. Das Bezugszeichen 2 bezieht sich in seiner Gesamtheit auf ein Gehäuse, innerhalb dem eine im Wesentlichen abgeschlossene Sterilisationskammer 4 zum Sterilisieren der Behältnisse ausgebildet ist.

Das Gehäuse wird dabei durch ein gewölbtes Bodenteil 12 und ein ebenfalls gewölbtes Deckelteil 16 ausgebildet. Durch diese jeweiligen Wölbungen wird erreicht, dass sich an diesen Oberflächen niederschlagendes Sterilisationsmittel nicht nach unten und dadurch gegebenenfalls in die Behältnisse tropft sondern seitlich - hier nach außen hin - ablaufen kann. Unterhalb der Behältnisse kann die Sterilisationsflüssigkeit aufgefangen werden und über eine Abführleitung 24 abgeführt werden. Entsprechend weist das Gehäuse einen schräg stehenden Boden 42 auf, der hier die Flüssigkeit in radialer Richtung nach außen drängt.

Bei der gezeigten Ausführungsform ist das gesamte Gehäuse stehend angeordnet. Es wäre jedoch auch denkbar, dass das Bodenteil 12 sich mit dem Träger mitdreht. In diesem Fall wären vorteilhaft zwischen dem stehenden Deckelteil 16 und dem drehenden Bodenteil 12 Dichtungseinrichtungen wie sog. aus dem Stand der Technik bekannte Wasserschlösser vorgesehen. In diesem Fall würde sich auch die Beaufschlagungseinrichtungen 6, welche in der Wandung 12a des Bodenteils 12 angeordnet sind, mit den Behältnissen 10 mitbewegen. Zu diesem Zweck wäre es weiterhin denkbar, Dreheinrichtungen vorzusehen, welche die Behältnisse während ihres Transports auch um ihre Längsachse drehen um so eine Beauschlagung der gesamten Umfangswandung der Behältnisse 10 zu erreichen. Das Deckelteil 16 bildet hier auch eine seitliche Wandung 16a aus, in der mehrere der Beaufschlagungseinrichtungen 6 angeordnet sind. Auch das Bodenteil 12 bildet eine Wandung 12a aus, in der eine Vielzahl von Beaufschlagungseinrichtungen 6 angeordnet ist.

Das Bezugszeichen 6 bezieht sich, wie gesagt, jeweils auf Beaufschlagungseinrichtungen, welche die im Inneren des Gehäuses bewegten Behältnisse 10 mit einer Sterilisationsflüssigkeit beaufschlagen. Dabei können diese Beaufschlagungseinrichtungen 6 beispielsweise als Sprühköpfe oder dergleichen ausgebildet sein, welche die Behältnisse, wie durch das Bezugszeichen S veranschaulicht, großflächig mit einem Desinfektions- bzw. Sterilisationsmedium beaufschlagen. Der untere Gehäuseteil bzw. die Sternsäule 12 bildet hier auch gleichzeitig eine Begrenzung der Sterilisationskammer 4. Innerhalb dieser relativ klein gehaltenen Sterilisationskammer kann sich eine Atmosphäre 30 aus Sterilisationsgas ausbilden, welche die Behältnisse besonders effizient sterilisiert. Die einzelnen Beaufschlagungseinrichtungen 6 können dabei in unterschiedlicher Weise auf die Behältnisse ausgerichtet sein, so dass nacheinander auch unterschiedliche Bereiche der Behältnisse sterilisiert werden. Auch wäre es möglich, Beaufschlagungseinrichtung 6 in dem Boden 42 vorzusehen.

Das Bezugszeichen 36 kennzeichnet grob schematisch eine außerhalb des Gehäuses angeordnete Zuführleitung, welche die Beaufschlagungseinrichtung 6 mit dem fließfähigen Medium versorgt. Dabei wäre es möglich, dass eine Zentralleitung vorgesehen ist, welche sich in eine Vielzahl der gezeigten Zuführleitungen aufteilt, um so eine Vielzahl von Beaufschlagungseinrichtungen 6 zu versorgen. Die Beaufschlagungseinrichtungen erstrecken sich hier jeweils durch die Wandungen 12a, 16a hindurch, sind also teilweise außerhalb des Gehäuses und teilweise innerhalb des Gehäuses 2 angeordnet. Auf diese Weise wird erreicht, dass die Beaufschlagungseinrichtungen innerhalb des Gehäuses nur einen sehr geringen Raum beanspruchen und so die Sterilisationskammer vergleichweise klein gestaltet werden kann. Vorzugsweise sind die Beaufschlagungseinrichtungen 6 gegenüber der Kammer justierbar so dass die Ausrichtung des Sprühkegels S der einzelnen Beaufschlagungseinrichtungen geändert werden kann. Bevorzugt ist daher eine Vielzahl von Beaufschlagungseinrichtungen 6 vorgesehen und ein Transportpfad der Behältnisse verläuft dabei zum Teil zwischen diesen Beaufschlagungseinrichtungen 6. Damit sind bevorzugt einige Beaufschlagungseinrichtungen radial innerhalb des Transportpfades der Behältnisse 10 angeordnet und einige Beaufschlagungseinrichtungen radial außerhalb dieses Transportpfades.

Figur 2 zeigt eine schematische Darstellung der in Figur 1 gezeigten Vorrichtung. Man erkennt hier auch ein Zuführrad 32, welches die Behältnisse an die Sterilisationsvorrichtung 1 zuführt sowie ein Abführrad 34, welches die sterilisierten Behältnisse wieder abführt. Das Bezugszeichen P kennzeichnet den hier kreisförmig ausgebildeten Transportpfad, entlang dessen die Behältnisse 10 transportiert werden. Das Zuführrad 32 und das Abführrad 34 sind hier so angeordnet, dass die Zu- und Abführung der Behältnisse 10 sehr nah aneinander liegen, so dass nur eine vergleichsweise kleine Öffnung in dem Gehäuse 2 vorgesehen sein muss. Durch diese Öffnung kann weiterhin dem Gehäuse auch beispielsweise auch im Rahmen von Wartungszwecken Sterilluft zugeführt werden, damit auf der anderen Seite über eine Abführeinrichtung 14 schnell das mit dem Sterilisationsmedium versetzte Gas aus der Anlage entnommen werden kann. Die Bezugszeichen 18 beziehen sich auf Öffnungen, welche beispielsweise zu Wartungszwecken geöffnet werden können, damit der Benutzer in die Maschine gelangen kann.

Die Vorrichtung weist weiterhin ein Reservoir für das Sterilisationsmedium auf, welches mit (nicht angezeigten) Zuführleitungen mit den einzelnen Beaufschlagungseinrichtungen 6 verbunden ist.

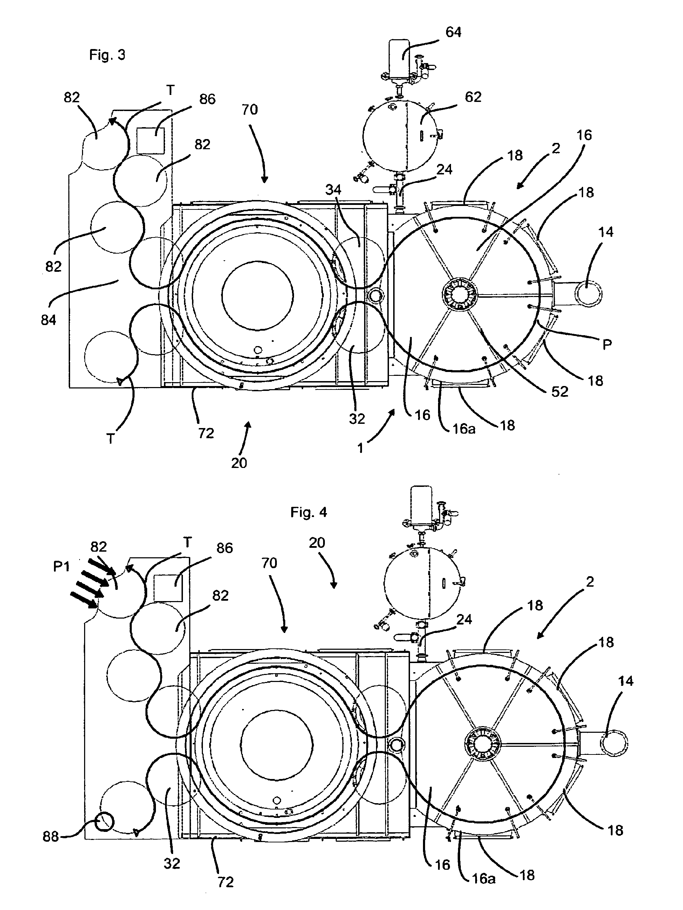

Fig. 3 zeigt eine weitere Draufsicht auf eine erfindungsgemäße Vorrichtung 1. Dabei ist hier insbesondere auch die Umgebung der Vorrichtung 1 dargestellt. Das Bezugszeichen T bezieht sich auf den gesamten Transportpfad, entlang dessen die Behältnisse durch die in ihrer Gesamtheit mit 20 bezeichnete Anlage transportiert werden. Der Transportpfad P ist ein Teil dieses Gesamttransportpfades T. An die Abführleitung 24 schließt sich ein Pufferbehälter 62 und eine Pumpe 64 an, um das Sterilisationsmedium aus der Vorrichtung 1 abzuführen. Das Bezugszeichen 52 kennzeichnet Versteifungen, welche das Deckelteil 16 der Vorrichtung 1 verstärken.

Das Bezugszeichen 70 kennzeichnet in seiner Gesamtheit eine Sterilisationseinrichtung zur Innensterilisation der Behältnisse. Dabei wäre es hier möglich, dass die Behältnisse mit einem Sterilisationsmedium beaufschlagt werden, es wären jedoch auch andere Arten der Sterilisation, beispielsweise über elektromagnetische Strahlung oder Elektronenstrahlung möglich. Das Bezugszeichen 72 kennzeichnet eine Wandung der Sterilisationseinrichtung 70. Die Bezugszeichen 82 beziehen sich jeweils auf Sternräder, welche die Behältnisse aus der Anlage abführen und das Bezugszeichen 84 auf einen Maschinenvortisch. Das Bezugszeichen 86 kennzeichnet eine Filterventilatoreinheit, die beispielsweise auf einem Isolatordach in der Art eines Zuluftsystems angeordnet sein kann.

Fig. 4 zeigt eine Darstellung ähnlich der in Fig. 3 gezeigten Darstellung, wobei hier jedoch zusätzlich entlang der Pfeile P1 der Luftstrom in die gesamte Anordnung 20 eingeleitet wird. In der Anlage mündet der Behältnistransportpfad T zu den stromabwärts folgenden Maschinen, wie beispielsweise einem Rinser, einem Füller, einem Verschließer oder einer Etikettiermaschine. Der besagte Luftstrom wird dann während der Produktion in der Figur unten links an der mit 88 gekennzeichneten Stelle bzw. dem Absaugstutzen 88 abgesaugt. Das bedeutet, dass während des Produktionsbetriebs der Luftstrom durch den Ein- und Auslaufbereich des Behältnissterilisators 70 und damit an der Vorrichtung 1 vorbeigeführt wird und somit nicht durch die Maschine verläuft. Sollte es zu einem Bedienereingriff kommen, so wird der Ansaugstutzen 88 vollständig oder teilweise geschlossen. Über geregelte Ventile im Lüftungssystem (nicht im Detail dargestellt) und eine Öffnung des Ansaugstutzens 14 wird nunmehr der Luftstrom auch durch den Behältnisinnensterilisator 70 und die Behandlungskammer der Vorrichtung 1 geleitet.

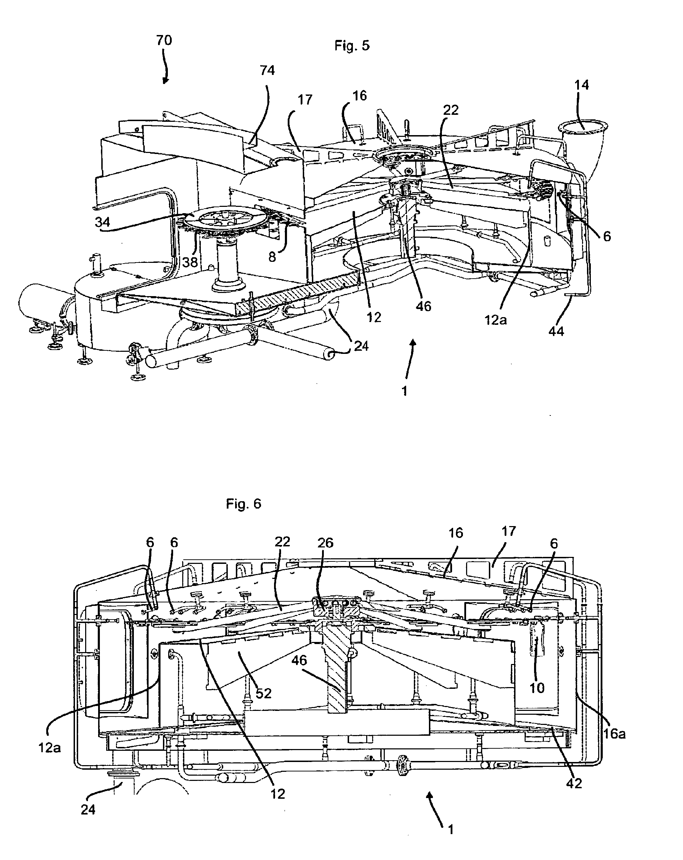

Fig. 5 zeigt eine Darstellung des Innenraums der Vorrichtung 1 in einer weiteren Ausführungsform. Hier erkennt man wiederum die einzelnen Ausleger 22 sowie das Bodenteil 12 der Vorrichtung 1. Das Bezugszeichen 44 bezieht sich auf eine Verrohrung, um das Sterilisationsmittel einzuführen. Das Deckelteil 16 ist mittels außen liegenden Versteifungen 17 versteift. Das Bezugszeichen 46 kennzeichnet eine Antriebseinrichtung, welche zum Transport der Behältnisse durch die Vorrichtung 1 dient. Die Antriebseinrichtung ist vorzugsweise als Direktantrieb ausgeführt, kann jedoch alternativ als jeder beliebige (elektro)motorische Antrieb ausgebildet sein. Das Bezugszeichen 24 kennzeichnet wieder die Abführleitung zum Abführen des flüssigen Sterilisationsmediums. Man erkennt, dass die Vorrichtung eine Vielzahl von Greifelementen 28 aufweist, welche jeweils zum Halten der Behältnisse dienen. Auch an dem Abführrad 34 sind entsprechende Greifelemente, wie Greifklammern 38 vorgesehen, welche die Behältnisse von der Vorrichtung 1 übernehmen. Das Bezugszeichen 74 kennzeichnet einen Ringkanal, der beispielsweise Bestandteil einer Ringkanaldichtung bzw. eines sogenannten Wasserschlosses zur Abdichtung des Innensterilisators 70 sein kann.

Fig. 6 zeigt eine weitere Darstellung des Innenraums der Vorrichtung 1. Man erkennt auch hier wieder die einzelnen Versteifungen 52. Weiterhin ist auch hier der schräge Boden 42 erkennbar, über den das Sterilisationsmedium abfließen kann. Auch ist die Schrägstellung des Deckels 16 erkennbar.

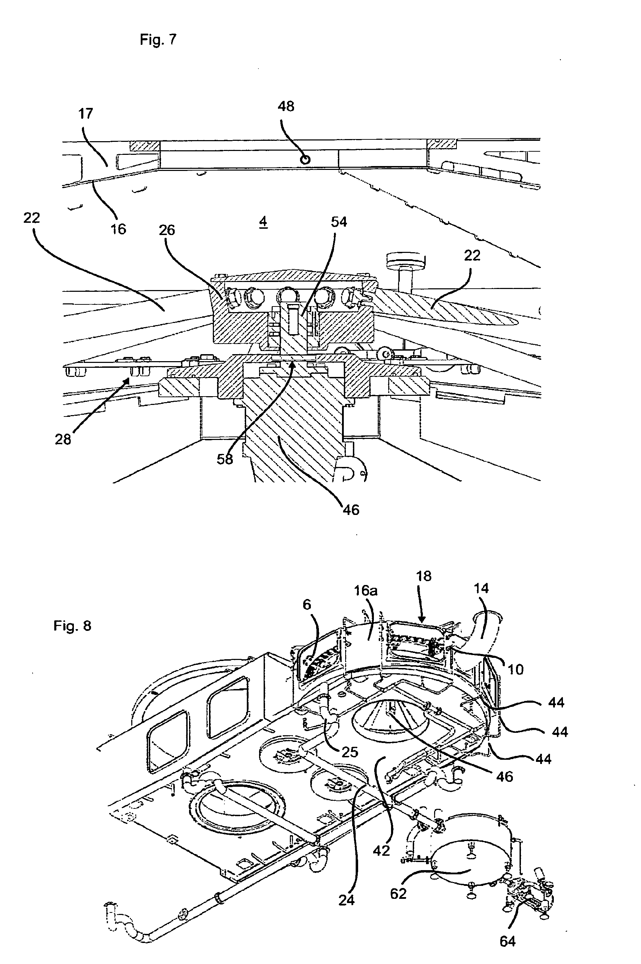

Fig. 7 zeigt eine weitere Detaildarstellung der in Fig. 6 gezeigten Vorrichtung. Das Bezugszeichen 54 bezieht sich auf die Nabe der Antriebseinrichtung 46. Das Bezugszeichen 58 kennzeichnet einen Dichtungsring. Das Bezugszeichen 28 in der Darstellung kennzeichnet ein Greifelement. Über eine Montageöffnung 48 wird eine Zugänglichkeit der Anlage möglich.

Fig. 8 zeigt eine weitere Darstellung der erfindungsgemäßen Anlage. Hier sind ebenfalls wieder gut die auch in Fig. 6 dargestellten Verrohrungen erkennbar sowie auch der Antrieb bzw. die Welle 46. Man erkennt, dass die Welle außerhalb der Sterilisationskammer 4 angeordnet ist. Weiterhin sind auch hier wieder die Abführleitungen 24 erkennbar, die zusätzlich einen Siphon 25 aufweisen.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 1: Vorrichtung
- 2: Gehäuse
- 4: Sterilisationskammer
- 6: Beaufschlagungseinrichtung
- 10: Behältnisse
- 12: Bodenteil, Sternsäule
- 12a: Wandung
- 14: Abführeinrichtung, Ansaugstutzen
- 16: Deckelteil
- 16a: Wandung
- 17: Versteifungen
- 18: Öffnungen
- 20: Anlage, Anordnung
- 22: Ausleger
- 24: Abführleitung
- 25: Siphon
- 26: drehbarer Träger
- 28: Greifelement
- 30: Atmosphäre,
- 32: Zuführrad
- 34: Abführrad
- 36: Zuführleitung
- 38: Greifklammer,
- 48: Montageöffnung
- 42: Boden
- 44: Verrohrung
- 46: Antriebseinrichtung, Welle
- 52: Versteifungen
- 58: Dichtungsring
- 62: Pufferbehälter
- 64: Pumpe
- 70: Sterilisationseinrichtung, Innensterilisator
- 72: Wandung
- 74: Ringkanal
- 82: Sternräder
- 84: Maschinenvortisch
- 86: Filterventilatoreinheit,
- 88: Absaugstutzen

- D: Drehachse
- S: Sprühbereich
- T: Gesamttransportpfad, Behältnistransportpfad
- P: Transportpfad
- P1: Pfeile

## Patentansprüche

1. Vorrichtung (1) zum Behandeln von Behältnissen (10) mit einem Gehäuse (2), einer innerhalb dieses Gehäuses (2) ausgebildeten Sterilisationskammer (4) zum Sterilisieren der Behältnisse (10), mit einer Transporteinrichtung (8), welche die Behältnisse (10) entlang eines vorgegebenen Transportpfades (P) durch diese Sterilisationskammer (4) transportiert, und mit wenigstens einer Beaufschlagungseinrichtung (6), welche die Behältnisse (10) während ihres Transports durch die Sterilisationskammer (4) mit einem fließfähigen Sterilisationsmedium beaufschlagt,
wobei die Beaufschlagungseinrichtung als Sprühkopf oder Düse ausgebildet ist **dadurch gekennzeichnet, dass**
die Beaufschlagungseinrichtung (6) wenigstens teilweise in eine Wandung (12) der Sterilisationskammer integriert ist und sich durch diese Wandung (12) hindurch erstreckt, derart, dass die Beaufschlagungseinrichtung (6) teilweise außerhalb und teilweise innerhalb des Gehäuses (2) angeordnet ist.

2. Vorrichtung nach Anspruch 1,
**dadurch gekennzeichnet**, das
die Sterilisationskammer (4) wenigstens abschnittsweise kanalartig um den Transportpfad (P) ausgebildet ist.

3. Vorrichtung nach wenigstens einem der vorangegangenen Ansprüche,
**dadurch gekennzeichnet, dass**
die Vorrichtung (1) eine Abführeinrichtung (14) zum Abführen eines gasförmigen Mediums aus der Sterilisationskammer (4) aufweist.

4. Vorrichtung nach Anspruch 2 und 3
**dadurch gekennzeichnet, dass**
die Abführeinrichtung (14) derart angeordnet ist, dass während des Absaugens eine gerichtete Strömung des gasförmigen Mediums durch die Sterilisationskammer (4) erreichbar ist.

5. Vorrichtung nach Anspruch 3,
**dadurch gekennzeichnet, dass**
die Vorrichtung eine Zuführeinrichtung für das gasförmige Medium aufweist und die Zuführeinrichtung von der Abführeinrichtung (14) beabstandet ist.

6. Vorrichtung nach wenigstens einem der vorangegangenen Ansprüche,
**dadurch gekennzeichnet, dass**
in dem Gehäuse (2) wenigstens eine verschließbare Montageöffnung (18) angeordnet ist

7. Vorrichtung nach Anspruch 1,
**dadurch gekennzeichnet, dass**
an dem Träger eine Vielzahl von Auslegern (22) angeordnet sind, an denen jeweils die Behältnisse (10) führbar sind.

8. Vorrichtung nach wenigstens einem der vorangegangenen Ansprüche,
**dadurch gekennzeichnet, dass**
ein Abschnitt der Transporteinrichtung (8) eine die Sterilisationskammer (4) abschließende Wandung aufweist.

9. Vorrichtung nach wenigstens einem der vorangegangenen Ansprüche,
**dadurch gekennzeichnet, dass**
die Sterilisationskammer einen unterhalb des Transportpfades (P) ausgebildeten Sammelraum zum Sammeln des fließfähigen Mediums aufweist.

10. Vorrichtung nach wenigstens einem der vorangegangenen Ansprüche,
**dadurch gekennzeichnet, dass**
das Gehäuse (2) kuppelartig ausgebildet ist.

11. Vorrichtung nach wenigstens einem der vorangegangenen Ansprüche,
**dadurch gekennzeichnet, dass**
die Vorrichtung (1) Zuführleitungen (36) aufweist, welche die Beaufschlagungseinrichtungen mit dem fließfähigen Medium versorgen und diese Zuführleitungen (36) im Wesentlichen außerhalb der Sterilisationskammer (4) angeordnet sind.

12. Vorrichtung nach wenigstens einem der vorangegangenen Ansprüche,
**dadurch gekennzeichnet, dass**
die Vorrichtung (1) eine Zuführöffnung (32) zum Zuführen der Behältnisse und eine Abführöffnung (34) zum Abführen der Behältnisse (10) aufweist.

## Claims

1. Apparatus (1) for treating containers (10), with a housing (2), a sterilisation chamber (4) formed within the housing (2) to sterilise the containers (10), with a transport device (8) which transports the containers (10) along a pre-specified transport path (P) through said sterilisation chamber (4), and with at least one actuating device (6) which acts upon the containers (10) during their transport through the sterilisation chamber (4) with a flowable sterilisation medium, wherein the actuating device is formed as a spray head or nozzle, **characterized in that** the actuating device (6) is at least partially integrated in a wall (12) of the sterilisation chamber and extends through said wall (12) in such a way that the actuating device (6) is arranged partially outside and partially inside the housing (2).

2. Apparatus according to claim 1, **characterised in that** the sterilisation chamber (4) is formed at least in sections channel-like about the transport path (P).

3. Apparatus according to at least one of the preceding claims, **characterised in that** the apparatus (1) has a discharge device (14) for discharging a gaseous medium from the sterilisation chamber (4).

4. Apparatus according to claim 2 or 3, **characterised in that** the discharge device (14) is arranged such that during extraction a targeted flow of the gaseous medium through the sterilisation chamber (4) can be achieved.

5. Apparatus according to claim 3, **characterised in that** the device has a supply device for the gaseous medium and the supply device is spaced from the extraction device (14).

6. Apparatus according to at least one of the preceding claims, **characterised in that** at least one closable mounting opening (18) is arranged in the housing (2).

7. Apparatus according to claim 1, **characterised in that** on the carrier is arranged a multiplicity of arms (22) on which respectively the containers (10) can be carried.

8. Apparatus according to at least one of the preceding claims, **characterised in that** a segment of the transport device (8) comprises a wall closing the sterilisation chamber (4).

9. Apparatus according to at least one of the preceding claims, **characterised in that** the sterilisation chamber has a collecting chamber formed below the transport path (P) to collect the flowable medium.

10. Apparatus according to at least one of the preceding claims, **characterised in that** the housing (2) is formed dome-like.

11. Apparatus according to at least one of the preceding claims, **characterised in that** the apparatus (1) has supply lines (36) which supply liquid medium to the actuating devices and these supply lines (36) are arranged substantially outside the sterilisation chamber (4).

12. Apparatus according to at least one of the preceding claims, **characterised in that** the apparatus (1) has a supply opening (32) for supply of the containers and a discharge opening (34) for discharge of the containers (10).

## Revendications

1. Installation (1) pour le traitement de récipients (10), avec une enceinte (2), une chambre de stérilisation (4) formée à l'intérieur de ladite enceinte (2) pour la stérilisation des récipients (10), avec un dispositif de transport (8) convoyant les récipients (10) au travers de ladite chambre de stérilisation (4) le long d'un chemin de transport (P) défini, et avec au moins un dispositif d'application (6), qui expose les récipients (10) à un milieu de stérilisation fluide pendant leur transport au travers de la chambre de stérilisation (4),
le dispositif d'application étant réalisé sous forme de tête de pulvérisation ou de buse,
**caractérisée en ce que**
le dispositif d'application (6) est au moins partiellement intégré à une paroi (12) de la chambre de stérilisation et **en ce qu'**il s'étend au travers de ladite paroi (12), de telle manière que le dispositif d'application (6) est en partie situé à l'extérieur de l'enceinte (2) et en partie à l'intérieur de celle-ci.

2. Installation selon la revendication 1,
**caractérisée en ce que**
la chambre de stérilisation (4) est au moins en sections réalisée sous forme de canal autour du chemin de transport (P).

3. Installation selon au moins une des revendications précédentes,
**caractérisée en ce que**
ladite installation (1) est pourvue d'un dispositif d'évacuation (14) pour l'évacuation d'un fluide gazeux hors de la chambre de stérilisation (4).

4. Installation selon les revendications 2 et 3,
**caractérisée en ce que**
le dispositif d'évacuation (14) est disposé de manière à permettre un écoulement dirigé du fluide gazeux au travers de la chambre de stérilisation (4) pendant l'aspiration.

5. Installation selon la revendication 3,
**caractérisée en ce que**
ladite installation est pourvue d'un dispositif d'amenée pour le fluide gazeux, et **en ce que** ledit dispositif d'amenée est espacé du dispositif d'évacuation (14).

6. Installation selon au moins une des revendications précédentes,
**caractérisée en ce qu'**
au moins une ouverture de montage (18) pouvant être fermée est prévue dans l'enceinte (2).

7. Installation selon la revendication 1,
**caractérisée en ce que**
plusieurs bras (22) sont disposés sur le support, contre chacun desquels les récipients (10) peuvent être guidés.

8. Installation selon au moins une des revendications précédentes,
**caractérisée en ce qu'**
une section du dispositif de transport (8) présente une paroi délimitant la chambre de stérilisation (4).

9. Installation selon au moins une des revendications précédentes,
**caractérisée en ce que**
la chambre de stérilisation est pourvue d'un compartiment collecteur formé sous le chemin de transport (P) pour la collecte du milieu fluide.

10. Installation selon au moins une des revendications précédentes,
**caractérisée en ce que**
l'enceinte (2) est en forme de coupole.

11. Installation selon au moins une des revendications précédentes,
**caractérisée en ce que**
ladite installation (1) est pourvue de conduites d'amenée (36) alimentant les dispositifs d'application en milieu fluide, et **en ce que** lesdites conduites d'amenée (36) sont disposées sensiblement à l'extérieur de la chambre de stérilisation (4).

12. Installation selon au moins une des revendications précédentes,
**caractérisée en ce que**
ladite installation (1) présente une ouverture d'amenée (32) pour l'amenée des récipients et une ouverture d'évacuation (34) pour l'évacuation des récipients (10).
